(19) **Europäisches Patentamt / European Patent Office / Office européen des brevets**

(11) **EP 1 711 636 B1**

(12) **EUROPEAN PATENT SPECIFICATION**

(45) Date of publication and mention of the grant of the patent:
**01.07.2009 Bulletin 2009/27**

(21) Application number: **05777709.6**

(22) Date of filing: **14.07.2005**

(51) Int Cl.:
*C12Q 1/68* (2006.01)

(86) International application number:
**PCT/EP2005/053384**

(87) International publication number:
**WO 2006/013144 (09.02.2006 Gazette 2006/06)**

(54) **METHOD OF IN-VITRO DETECTION AND QUANTIFICATION OF HIV DNA BY QUANTITATIVE PCR**

VERFAHREN ZUR IN-VITRO DETEKTION UND QUANTIFIZIERUNG VON HIV DNA MITTELS QUANTITATIVER PCR

PROCEDE DE DETECTION ET NUMERATION IN-VITRO DE L'ADN DU VIH PAR PCR QUANTITATIVE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **06.08.2004 ES 200401986**

(43) Date of publication of application:
**18.10.2006 Bulletin 2006/42**

(73) Proprietor: **Geno-Madrid S.A.**
**28004 Madrid (ES)**

(72) Inventors:
• **MUÑOZ FERNANDEZ, Maria Angeles**
**28017 Madrid (ES)**
• **FERNANDEZ GOMEZ-CHACON, Geronimo**
**28005 Madrid (ES)**

(74) Representative: **Illescas, Manuel et al**
**GONZÁLEZ-BUENO & ILLESCAS**
**Calle de Recoletos, 13 - 5º Izq.**
**28001 Madrid (ES)**

(56) References cited:
**EP-A- 0 630 973       US-B1- 6 294 326**

• **DATABASE WPI Section Ch, Week 200037 Derwent Publications Ltd., London, GB; Class B04, AN 2000-425919 XP002364895 -& JP 2000 139500 A (ZH TOKYOTO RINSHO IGAKU SOGO KENKYUSHO) 23 May 2000 (2000-05-23)**
• **DAMOND FLORENCE ET AL: "Quantification of proviral load of human immunodeficiency virus type 2 subtypes A and B using real-time PCR" JOURNAL OF CLINICAL MICROBIOLOGY, vol. 39, no. 12, December 2001 (2001-12), pages 4264-4268, XP002364889 ISSN: 0095-1137**
• **ZAZZI M ET AL: "SIIMULTANEOUS AMPLIFICATION OF MULTIPLE HIV-1 DNA SEQUENCES FROM CLINICAL SPECIMENS BY USING NESTED-PRIMER POLYMERASE CHAIN REACTION" AIDS RESEARCH AND HUMAN RETROVIRUSES, NEW YORK, NY, US, vol. 9, no. 4, 1993, pages 315-320, XP001019662 ISSN: 0889-2229**
• **GIBELLINI DAVIDE ET AL: "Quantitative detection of human immunodeficiency virus type 1 (HIV-1) proviral DNA in peripheral blood mononuclear cells by SYBR green real-time PCR technique." JOURNAL OF CLINICAL VIROLOGY : THE OFFICIAL PUBLICATION OF THE PAN AMERICAN SOCIETY FOR CLINICAL VIROLOGY. APR 2004, vol. 29, no. 4, April 2004 (2004-04), pages 282-289, XP002364890 ISSN: 1386-6532**

**Description**

FIELD OF THE INVENTION

The invention relates to the field of PCR-based methods and devices for diagnosis. Concretely, it relates to the detection and quantification of HIV DNA in patients.

STATE OF THE ART

The main parameters used for monitoring HIV infection are the plasma "viral load" (quantification of HIV RNA) and the percentage or number of CD4 lymphocytes [1]. It is very important to know the number of copies of HIV DNA present in cells, especially in patients undergoing Highly Active Antiretroviral Therapy (HAART) in which the viral load has decreased, even to an undetectable level, and an increase or stabilization of the percentage or number of CD4 lymphocytes has been observed, as well as in individuals when the treatment is interrupted [3].

Benefits of quantification of viral DNA:

- Diagnosis of HIV infection in children of seropositive mothers and in adults during the window (when treatment is interrupted). The advantage is that, being quantitative, it will give the number of copies of HIV DNA present in the cells at the time closest to infection, which can be converted to a prognostic marker of the development of infection.
- In patients treated with highly active antiretroviral therapy (HAART), in which the viral load is undetectable (< 400 copies/ml; ultrasensitive < 50 copies/ml), at present the CD4 lymphocytes are the only existing immune system marker, it would be very important to possess a virological marker and in this sense it would be possible to use the number of copies of HIV DNA present in the cells as a prognostic marker of development, which predicts when a jump in viral load is going to occur and avoid the appearance of resistant strains.
- In patients when treatment is interrupted. Before the antiretroviral treatment is interrupted, one of the requirements that the patient must satisfy is an undetectable viral load, so it would be very useful to know the number of copies of HIV DNA present in the cells prior to interruption and to know how long they can remain without treatment so there is no jump in viral load and development of resistance to the antiretrovirals.
- In patients for whom an immunological treatment or therapeutic vaccine is to be applied, where it would be very important to know the number of copies of HIV DNA present in the cells in order to see up to what point the number of cells infected is important for achieving an immunological system that is functional in the long term and which can control the replication of HIV by itself.
- To detect infected cell subpopulations that can act as a reservoir of HIV and that potentially cannot be infected by it.
- Application to basic research in various studies in vitro and in vivo.
- As a parameter for the detection and monitoring of inactive HIV.
- In general it will be a very useful technique for application in routine clinical practice and in clinical tests of HIV+ patients.

The application of new antiretroviral therapies to HIV infection has caused dramatic changes in the natural development of this infection, therefore it is very interesting to know the relationship between reconstitution of the immune system and persistence of HIV in the patient, moreover we would be able to find out at what moment the immune system would be able to control the infection and antiretroviral treatment can be withdrawn. The existing techniques of quantification of viral load and genotyping or phenotyping are not able to predict the development of the infection when the viral load is undetectable independently of the percentage or number of CD4 T lymphocytes.

The quantification of viral DNA proposed by the present invention represents a novel technique which could be useful as a diagnostic and prognostic marker of development of the infection and for monitoring it. In addition, it would enable us to detect the cellular subpopulations that act as a reservoir for HIV.

We present an innovative method for quantifying HIV DNA (viral DNA load) in infected cells, in a simple, reproducible and rapid manner by quantitative PCR in real time. The method described detects and quantifies all the forms of HIV DNA present in the cell, both integrated and non-integrated, even though there is a very small number of copies, applying in this case an embodiment of the invention that makes the method ultrasensitive. At the present time there is no method that is capable of detecting proviral HIV DNA (integrated into the genome) in a specific, reproducible, simple and rapid manner.

The ultimate objective of any living organism is its perpetuation in time. Like other retroviruses, the only way that HIV has of achieving this is to insert its genome in the cellular DNA.

In the first stages of the infection cycle, at the end of the uncoating process, the reverse transcription reaction begins, giving rise to the Pre-Integration Complex (PIC), with the viral genome now in the form of DNA, which moves to the nucleus. There, the PIC can undergo various processes: recircularization of the viral DNA with one or two long terminal repeats (LTR), or integration into the cell chromosome [2]. It is only in this last case that the functional provirus, the only infectious form, is established. In this way, the HIV will not be detected by the immune system and will be able to remain in the cell, and it will replicate with the rest of the cellular DNA, generating new cells carrying HIV. In optimum conditions this HIV DNA will be transcribed and translated by the cellular machinery, generating new HIV particles which will infect new cells [2].

At present it is possible to quantify the number of viral particles circulating in plasma by the "viral load" technique (detection of HIV RNA) which, together with the immunological marker for quantification of CD4 lymphocytes, are the two parameters usually employed for monitoring HIV infection in patients by medical personnel, for introducing, or changing, antiretroviral treatment.

The number of viral particles that can infect the same cell is variable, as is the number of times that the same RNA is reverse transcribed, so that the amount of viral DNA contained in a cell is variable, and there may be a very large number of copies or a very small number of them.

The proviral DNA represents a hidden threat to the life of the cell, and therefore to the life of the infected person, because the viral load can be activated and can increase without this being predicted by any of the parameters used, leading to failure of the antiretroviral therapy and emergence of resistant strains. For this reason, in patients who are beginning antiretroviral treatment for the first time, or are being treated with HAART, some other therapy, or beginning interruption thereof, it may be important to know the behaviour and variations of the proviral DNA, *"Proviral Load"*.

Various tests have been developed in recent years for the detection and quantification of proviral DNA using the PCR (polymerase chain reaction) technique, but these methods have not been able to detect it in all cases [4] [5] [6] since they only operate with indications of the position at which the viral DNA is integrated into the cellular genome. The sequence of the HIV genome is now well known and therefore it has been possible to design pairs of primers or oligonucleotides which form rings in some gene of the HIV and thus its presence can be detected by PCR and the total DNA can be quantified.

Methods are described in the state of the art for the detection of HIV DNA by PCR, by designing primers that bind in the *pol* viral gene and in the *gag* gene, as they are the genes that have a less variable sequence. Concretely, the *gag* gene has 1300 bp, from which it is possible to obtain hundreds or thousands of pairs of different primers, each one with characteristics that are also different, in particular on the basis of their "melting point" (Tm). Some techniques use simple PCR with primers of *pol* or *gag,* or alternatively "nested PCR", that is, two consecutive PCRs with the same or a different pair of primers for each, using the product amplified in the first as the DNA template for the second. Detection of the product in these two types of PCR is achieved by visualization of the amplified product in agarose gel. Consequently, the information obtained is only qualitative, not quantitative, in contrast to the method of our invention.

Other methods propose quantifying the PCR product by ELISA or hybridization on the amplified product after carrying out electrophoresis in agarose gel.

Furthermore, the document Yourno et al., J. Clinical Microbiol. 30, 2887-2892 (1992) discloses a method for detection of HIV in blood samples. The method is based on nested PCR using gag primers and an internal probe.

The method of the invention uses the technique of quantitative PCR in real time from Roche "LightCycler-qPCR" (LC-qPCR), although it would be possible to use any system of quantitative PCR (Applied Bio-System), with appropriate adaptation of the amplification programme, reference curves and other parameters known by any person skilled in the art who uses methods of quantitative PCR of this type. For the method of the invention, primers were designed for the *gag* gene, and a pair of probes of the F.R.E.T. type (Fluorescence Resonance Transfer) for detecting the amplified DNA product. This permits greater accuracy in detection of the specific product, without detecting possible undesirable by-products, since it is necessary for said probes to bind in their exact positions of the product for it to be quantified, in contrast to the Taqman® probe, which when acting on request, can quantify products not specific to circle formation in products with sequence similar to their target. To date, no method has been described for quantification of proviral DNA of HIV using the LightCycler-qPCR system of quantitative PCR.

one object of the present invention is to develop pairs of oligonucleotides which bind specifically to the *gag* gene of the HIV virus and permit amplification of the viral DNA by PCR.

A second object of the invention is to develop a pair of probes which permits efficient detection, by fluorescence, of the product of viral DNA of HIV amplified by PCR, eliminating the nonspecific background.

A third object of the invention is a method of in-vitro detection of viral DNA of HIV which permits the detection of proviral DNA of HIV.

Another object of the invention are test kits which comprise the oligonucleotides of the invention, optionally also using the probes of the invention.

A preferred embodiment of the invention comprises applying the method of detection also to samples with low viral load (<100 copies).

Another preferred embodiment of the invention comprises modifying the method of detection and quantification of HIV DNA by developing a 2nd pair of primers, which makes it possible to shorten the test times while at the same time increasing the specific fluorescence signal of the viral DNA present in the test sample.

A further object of the invention comprises the use of the oligonucleotides and the probes, in kits and/or methods of detection of viral DNA of HIV.

Description of the invention

The invention comprises an innovative method for the quantification of HIV DNA in cells. This method is characterized by sensitivity, reproducibility and specificity for the detection/quantification of HIV DNA in samples with a medium/high number of initial copies and moreover a preferred embodiment of the invention comprises an ultrasensitive method for the detection of samples with low initial copies.

An important point that should be emphasized in this method is the extraction of the DNA, which must be obtained in pure and clean form, and its quantification by UV spectrophotometry in order to find out the amount of template to be used in the PCR reaction, thus permitting standardization of the results per cg of DNA or per $10^6$ cells.

It is not possible to assess a *priori* whether the amount of HIV DNA present in each sample can be detected by the conventional method of the invention or whether it is necessary to have recourse to the ultrasensitive method. For this reason, LC-qPCR-Gag is carried out first, and if amplification is not obtained, the ultrasensitive method for samples with low number of initial copies of HIV DNA is applied. The ultrasensitive method for samples with low number of initial copies of HIV DNA is only applied directly in the case when the number of CD4 lymphocytes is below 400 cells/ml.

Only the T cells present in the mononuclear cells of peripheral blood of the patient are potential carriers of the viral DNA and, in accordance with the information obtained by this method, the number of copies of viral DNA per T lymphocyte varies in relation to the number of CD4 lymphocytes, therefore the quantification of the HIV DNA must be analysed in comparison with the number of CD4 lymphocytes and the viral load to permit better assessment of development of the infection.

To apply the method of the invention, the quantitative PCR apparatus called LightCycler (Roche) is used, which comprises a thermal cycler with air cooling and heating of the samples. This apparatus is connected to a fluorometer which detects the fluorescence emitted, for example that can be attributed to probes of the FRET type that bind to the product formed in each cycle of the polymerase chain reaction (PCR). For this apparatus to give a number of copies or counts in the samples it must extrapolate the fluorescence obtained in these to a reference straight line or curve that is obtained by putting a known number of DNA copies at each point on said straight line. The apparatus, connected to a computer, relates the fluorescence obtained at each point on the curve to the respective number of copies of DNA and shows it on a graph of fluorescence versus number of copies, this graph being adjusted to a straight line that enables us to calculate, on the basis of the fluorescence measured in the test sample, the number of copies of viral DNA present in the latter.

For detecting the HIV DNA by PCR we require a pair of primers, oligonucleotides or initiators which bind in some viral gene, and use an appropriate programme [8]. HIV is a retrovirus with high capacity for mutation, mainly at the *env* gene and, in the presence of antiretroviral treatment, also at the *pol* gene. For this reason the initiators must bind in more conserved genes, with less variability, such as the *gag* gene, thus making it possible to detect all the possible variants of HIV, in all the forms present in the cell, integrated or not integrated.

For this innovative method, two primers have been designed, F-Gag and R-Gag (SEQ ID NO: 1 and SEQ ID NO: 2), which amplify a 222 bp fragment of the *gag* gene (at positions 1260 to 1482) since, as already mentioned, it is a gene with a highly conserved sequence. Throughout this specification, the terms primer, initiator or oligonucleotide are used as synonyms.

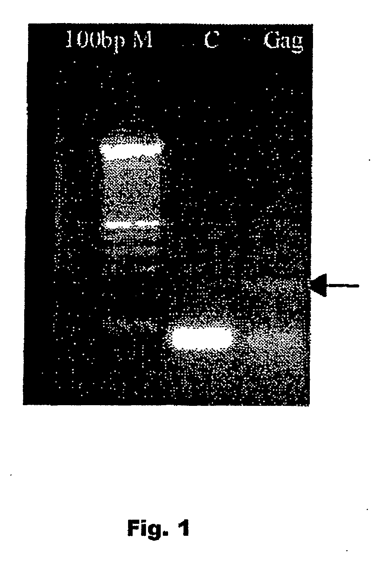

To determine the best conditions of the programme that has to be applied for carrying out the PCR, various experiments were repeated, varying the temperature of circle formation of the primers, their concentration, the concentration of $MgCl_2$ and the number of cycles. The conditions selected were: a programme that has initial denaturing of 10 minutes at 95°C, followed by 35 cycles of amplification with 1 minute at 95°C, 30 seconds at 65°C and 1 minute at 72°C, with a final extension of 4 minutes at 72°C. Each 25 µl of reaction mixture contains 0.5 to 1 µg of genomic DNA, 0.8 µM of each primer, 0.10 mM dCTP, 0.10 mM dGTP, 0.10 mM dATP, 0.10 mM dTTP, 1 U of DNA polymerase enzyme Amplitaq Roche, 50 mM KCl, 3.5 mM $MgCl_2$ and 10 mM of Tris-HCl (pH 8.3). The amplified product was tested in 2% agarose gel (Fig. 1).

A preferred embodiment of the invention comprises modifying the method, carrying out nested PCR using, in addition to the 1st pair of primers (SEQ ID NO: 1 and 2) in a 1st amplification, a 2nd pair of primers F2-Gag and R2-Gag (SEQ ID NO: 7 and 8), which amplifies in its turn, in a 2nd stage, the amplified product resulting from the 1st stage of amplification (product Gag1). This 2nd pair of primers binds inside the fragment amplified by the 1st pair (between positions 1286 and 1435), giving a product Gag2 of 149 bp, on which the probes P1-Gag and P2-Gag bind (between positions 1320 and 1381).

Detailed description of the invention

Example 1: Quantitative PCR-Gag with the LightCycler system

The genomic DNA was extracted from $10^6$ cells with the "Wizard SV Genomic DNA purification system" kit from PROMEGA (cat. #A2360), and resuspended in 100 $\mu$l of nuclease-free water PROMEGA (cat. #P119C). Finally, this DNA was quantified with the *Nano-Drop 3.0.0 /3* spectrophotometer.

The equipment for carrying out the quantitative PCR is a LightCycler, a thermal cycler using air which incorporates a fluorometer for the detection and quantification of the amplified products without the need for post-PCR analysis in agarose gel of the products obtained, as occurs when carrying out conventional PCR. Amplification was carried out in glass capillaries using a modified DNA polymerase enzyme of high efficiency, high rate of elongation and capacity for supporting rapid temperature changes (20°C/s). The advantage of using glass capillaries is that it permits rapid heating and cooling of the reaction mixture, and therefore very short cycles and programmes are carried out [7] [9]. The amplified products are detected by SYBR-Green, a marker compound that binds to double-stranded DNA by intercalation and emits fluorescence when excited with light of a suitable wavelength. Another means of detection/quantification is the use of DNA probes (SEQ ID NO: 5 and SEQ ID NO: 6) labelled with fluorochromes (F.R.E.T. type), which bind specifically to the amplified product. The products are quantified by extrapolation of the fluorescence obtained in each of the samples relative to a reference curve obtained with a known number of copies.

Example 2: Detection of the amplified product by SYBR-Green (Cambrex)

This embodiment of the invention is used for samples whose viral DNA load is assumed to be high. At the start of development of the method of quantification of HIV DNA, conventional PCR-Gag was adapted to the conditions of the LightCycler quantitative PCR (LC-qPCR). The programme for the new thermal cycler begins with 10 minutes of denaturing at 95°C, followed by 40 cycles with 15 seconds of denaturing at 95°C, 10 seconds for circle formation of primers and single data collection of fluorescence, and 20 seconds of elongation at 72°C. Each 20 $\mu$l of reaction mixture contains 0.5 $\mu$g to 1 $\mu$g of genomic DNA, 0.5 $\mu$M of each primer (F-Gag and R-Gag), 0.10 mM of dATP, 0.10 mM of dCTP, 0.10 mM of dTTP, 0.10 mM of dGTP, and 1 U of LightCycler FastStart DNA polymerase enzyme (Roche), 50 mM KCl, 3.5 mM $MgCl_2$, and 10 mM Tris-HCl (pH 8.3). The amplified products are detected with SYBR-Green. This marker only detects double-stranded DNA, therefore it does not interfere with the primers used, unless they form dimers. The reference curve was obtained using serial dilution in steps of an order of magnitude from $10^4$ to 1 copy per reaction mixture, with DNA from cells of the 8E5 line, a lymphoid cell line that is characterized in that it carries a single HIV provirus per cell. To produce this reference straight line or curve, the DNA is extracted from 10 aliquots with 1 million cells per aliquot, with measurement or quantification in a UV spectrophotometer. The average weight of the genomic DNA obtained is found and the weight of the genomic DNA of an 8E5 cell is determined at an average of 5pg, meaning that 5pg of DNA corresponds to one copy of HIV DNA.

The system for detecting the amplified product by SYBR-Green is nonspecific, since it binds to all the double-stranded DNA present in the reaction mixture, for example the dimers of primers, therefore producing much background and generating some quantification data with errors. Although the computer program of the LightCycler-qPCR system makes it possible to subtract the background fluorescence obtained, negative control of the remaining samples, a significant error would be made, since the background due to the dimers of primers is different in each sample, depending on their initial amount of template, larger amount of initial template leading to less formation of dimers of primers, therefore it is not possible to subtract the same amount of fluorescence from each sample. However, this error is acceptable in the case of samples with a high viral DNA load.

Example 3: Detection of the amplified product by means of F.R.E.T. probes

So as to be able to avoid these problems in the quantification of HIV DNA, when the viral DNA load is not high or is not assumed to be high, a pair of DNA probes labelled with fluorochromes was designed, of the F.R.E.T. type, designated P1-Gag and P2-Gag (SEQ ID NO: 5 and SEQ ID NO: 6), which hybridize specifically in a region between the F-Gag and R-Gag primers of the product amplified in PCR-Gag. In this way only the specific PCR product without a background signal, and being real data, is detected and quantified.

To improve and increase the accuracy of the method of detection with SYBR-Green, a new reference curve was constructed that is more exact than the previous one with DNA from 8E5 cells. To construct this reference curve, the product amplified with conventional PCR-Gag, with DNA from the 8E5 cell, was cloned in the targets EcoRI (SEQ ID NO: 4) and BamHI (SEQ ID NO: 3) within the plasmid Bluescript II(-) (pBSK II-), Stratagene #212216, grown in *Escherichia coli DH5a* bacteria and purified by Maxiprep. The plasmid with the insert was designated "pBSKGag" and after quantifying it in a UV spectrophotometer and determining the number of molecules of plasmid per $\mu$l, a serial dilution

was prepared from it with steps of the order of magnitude from $10^{10}$ to 1 molecule/$\mu$l. The primers shown as SEQ ID NO: 3 and SEQ ID NO: 4 are used for cloning the amplified product between the targets BamHI and EcoRI of the plasmid pBSK(-) for use as template DNA in the reference straight line. These primers shown by SEQ ID NO: 3 and SEQ ID NO: 4 are the same as SEQ ID NO: 1 and SEQ ID NO: 2 but with the bases added at 5' corresponding to the targets of the restriction enzymes described. Each 20 $\mu$l of reaction mixture of LC-qPCR-Gag contains from 0.5 to 1 $\mu$g of genomic DNA as template, 0.2 $\mu$M of each fluorescent probe P1-Gag and P2-Gag, 0.5 $\mu$M of each primer F-Gag and R-Gag, 0.10 mM of dATP, 0.10 mM of dCTP, 0.10 mM of dTTP, 0.10 mM of dGTP, and 1 U of LightCycler FastStart DNA polymerise enzyme (Roche), 50 mM KCl, 3.5 mM MgCl$_2$, and 10 mM Tris-HCl (pH 8.3).

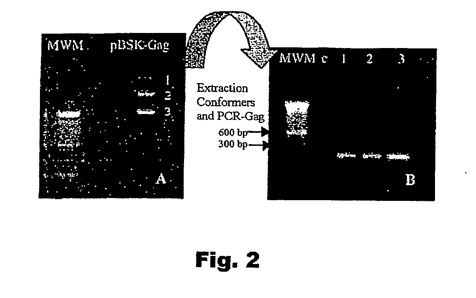

With the aim of verifying the exactness of the reference curve and ensuring that all the conformers of plasmid pBSK-Gag present and quantified by UV spectrophotometry in said curve give the expected product in said PCR, electrophoresis was carried out in 1% agarose gel (Fig. 2), with 500 ng of said plasmid to observe its various conformers: linear, circular relaxed (with a cut in one of its strands) and supercoiled. The three conformers were cut out of the gel and extracted separately with GENECLEAN® Spin kit - BI0101 (Cat. #1101-200) in 20 $\mu$l of nuclease-free water. 5 $\mu$l was collected as template from each one for PCR-Gag and in order to see the result 10 $\mu$l of said PCR was loaded in a 2% agarose gel. The Gag product was obtained from the three conformers, which clearly indicates that all the plasmid pBSK-Gag quantified gives product in PCR-Gag. In conclusion, the theoretical DNA copies of the reference curve are very close to the real ones.

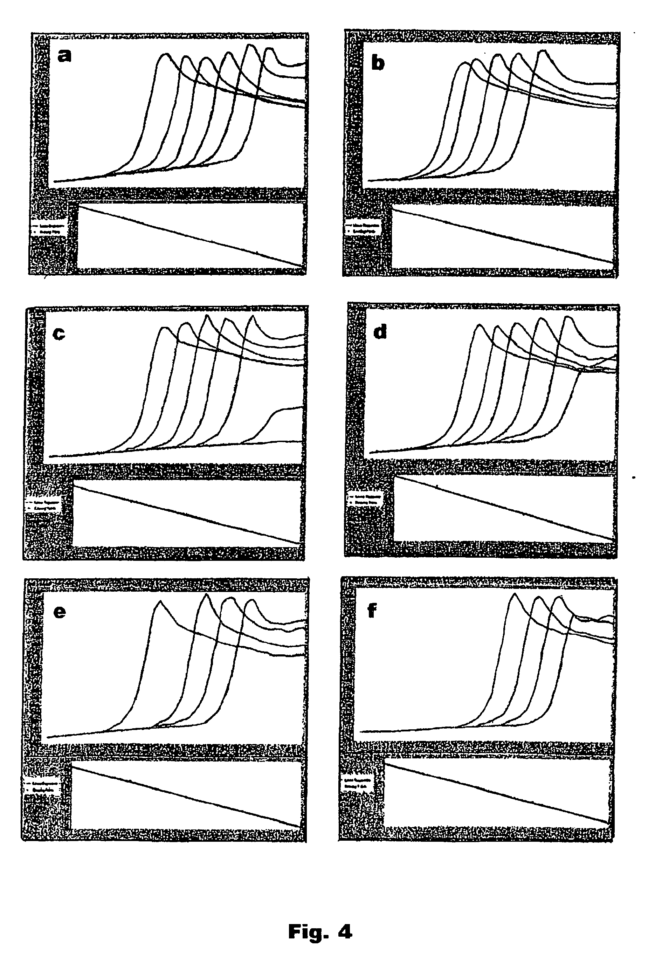

We deduce, from the results obtained in various tests with the reference curve (Fig. 4), that it is possible to take at least 4 points, from the total of 7. The average rate of variation between the different reference curves is less at the points from $10^8$ to $10^5$ (from -0.1 to +0.1) than at the points $10^4$ and $10^3$, -0.2 to +0.2, and at point 100 it is -3.5. The remaining points 10 and 1 have an average rate of variation of -59.1 and -319.0 respectively, too high, since they have problems of amplification associated with the samples with target DNA at low number of copies; therefore these points are never included in the real reference curve, so that the lower limit of detection with this qPCR (Table 1) would be 100 copies.

Table 1: Reference curves pBSK-Gag of LC-qPCR Gag.

| Tube | Theoretical number of copies | Exp1: 18-3-04 | Exp2: 20-2-04 | Exp3:20-2-04b | Exp4: 23-2-04 | Exp5: 23-2-04b | Exp6: 2-3-2004 | Average rate of variation |
|---|---|---|---|---|---|---|---|---|
| 1 | 100000000 | 112500000 | 131100000 | 97580000 | 102400000 | 104000000 | | -0.1 |
| 2 | 10000000 | 8339000 | 12390000 | 10970000 | 9607600 | 13100000 | | -0.1 |
| 3 | 1000000 | 1055000 | 1180000 | 895000 | 1393000 | 965000 | 1133000 | -0.1 |
| 4 | 100000 | 98600 | 74160 | 1040001 | 96600 | 92000 | 87300 | 0.1 |
| 5 | 10000 | 10070 | 4739 | 7300 | 5946 | 10800 | 9028 | 0.2 |
| 6 | 1000 | 1018 | 874 | 16561 | 839 | 1900 | 1120 | -0.2 |
| 7 | 100 | 222 | 542 | 795 | 150 | 568 | 431 | -3.5 |
| 8 | 10 | 600 | 602 | | | | | -59.10 |
| 9 | 1 | 250 | 390 | | | | | -319.00 |
| NEG control | 0 | 0 | 0 | 0 | 0 | 0 | 0 | |
| H2O control | 0 | 0 | 0 | 0 | 0 | 0 | 0 | |
| | | | | | | | | |
| Internal C 1 | 100000 | | | 1070001 | | | | |
| Internal C 2 | 1000 | 10781 1 | 1177 | 15871 | | | | |
| R | | 1 | 1 | 1 | 1 | 1 | 1 | |
| Error | | 0.05 | 0.1 | 0.07 | 0.1 | 0.04 | 0.08 | |

Standard Average error of the reference curve: 0.07 Exp1-5: Experiments on different dates.

C1 and C2 are internal controls which are incorporated in the reference straight line without this being indicated in the programme

Example 4: Detection of the product amplified in samples with low number of copies. Nested PCR or double PCR using the same pair of primers twice in succession.

Quantification in samples with low number of copies in the template DNA is very difficult because copies may not be detected or a higher number of copies may be detected than are really present in the sample. The formation of dimers of the primer and nonspecific amplification products are problems associated with samples of this type, since they are generated more easily than the product of interest. These amplification artefacts compete with the target sequences for the reagents of the PCR reaction mixture, giving abnormal values of amplification and quantification.

To increase the number of copies in the template of LC-qPCR, we carry out a "nested PCR" or double PCR, a combination of conventional PCR-Gag and LC-qPCR-Gag, using the same primers in the two PCRs, F-Gag and R-Gag. In this way it is possible to detect the target DNA when there is a very small amount in the sample. The first conventional PCR-Gag makes it possible to generate sufficient product from the initial sample, for use subsequently as template in LC-qPCR-Gag. In this way there is sufficient starting target DNA without the artefacts or dimers of the primer competing for the specific product of PCR, thus permitting its correct quantification.

The template used for carrying out LC-qPCR is 10 $\mu$l of the 25 $\mu$l of the conventional PCR-Gag of samples from patients, and three points are put on the reference curve with low number of copies: 100, 10 and 1 copy per reaction mixture. The quantification obtained in LC-qPCR corresponds to the number of copies present in the 10 $\mu$l of conventional PCR used as template in LC-qPCR, the real copies do not exist in the sample. Using the counts obtained at each of the points on the curve with 100, 10 and 1 initial copy, the efficiency (E) of conventional PCR-Gag is obtained using the equation: [8]

$$\boxed{Tn = To(E)^n} \quad (1)$$

where $Tn$ is the number of copies at the end of PCR, $To$ is the number of initial copies in the template, $E$ is the efficiency of the PCR reaction (it is usually between 1.8 and 2), and n is the number of cycles of PCR. The point on the reference curve 100 has To(100) = 100 and its Tn(100) in conventional PCR-Gag is its To(100)q in LC-qPCR-Gag. Inserting these values in equation (1), we get the value of E:

$$E = \sqrt[n]{Tn/To} \quad (2)$$

$$E = \sqrt[35]{To(100)q/100} \quad (3)$$

The E obtained in (3) of conventional PCR-Gag is calculated for the three points of the reference curve, low-10-1, and the *average E (1.8-2)* is determined, which can be assumed to be the same for all the samples of the same reaction, so that on this basis it is possible to determine the number of initial copies present in patients' samples, To(pi), (4). The E of a PCR is not exactly the same for all the samples, nor in all of the reaction cycles, since it depends on the number of target DNA present in the template. In the initial cycles, the template is small, and so is E, and as the amount of template increases so too does E, since there is more template and more copies are produced, amplification becoming exponential, therefore E approaches 2.

In LC-qPCR-Gag, for each patient sample the To(pi)q is obtained, which is the Tn(pi) from conventional PCR-Gag, which when inserted in (4) gives the To(pi).

$$To\ pi = Tn\ pi\ /\ E^{35} \quad (4)$$

The index of E depends on the number of PCR cycles applied.

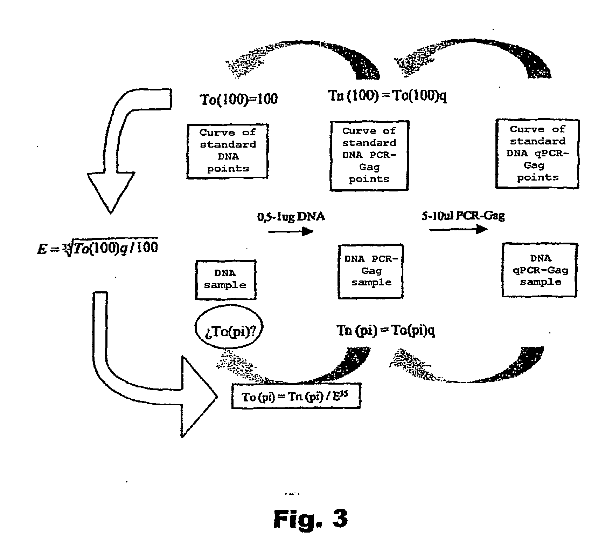

The algorithms adopted constitute the computer program designed and included in the present invention for correct application of the method of measurement of viral DNA described (explanatory diagram in Fig. 3).

Example 5: Results of application of the Gag1 ultrasensitive method

The technique described was applied to a group of HIV infected patients undergoing HAART therapy with low or undetectable "viral load". LC-qPCR-Gag was carried out, without obtaining counts in any of them, therefore the method was applied for samples with low number of initial copies, first carrying out a conventional PCR-Gag, and then 10 $\mu$l of this is used as template for LC-qPCR-Gag.

The results obtained in these samples were below 435 copies/$\mu$g DNA (1306 copies/$10^6$ cells), there being samples with less than 1 copy/$\mu$g DNA, i.e. 1 copy per 333333 cells (since the genome of a human cell is approximately equivalent to 3pg). Only in the cases in which amplification was not obtained is it possible to say that they have "undetectable HIV DNA".

Samples with medium/high values of HIV DNA are detected in the first LC-qPCR-Gag and are between 1874 and 3367 copies/$\mu$g DNA (from 5623 to 10100 copies / $10^6$ cells). (Tables 2 and 3).

Table 2: Result from samples with medium/high number of copies

| Sample | N cop qPCR | Vol template($\mu$l) | vol DNA 1 million cells($\mu$l) | N cop/million cells | Ncop/$\mu$g DNA |
|---|---|---|---|---|---|
| E 3.0 | 562.3 | 10 | 100 | 5623 | 1874 |
| E 3.4 | 761.4 | 10 | 100 | 7614 | 2538 |
| E 4.0 | 513.3 | 10 | 100 | 5133 | 1711 |
| E 4.4 | 1010 | 10 | 100 | 10100 | 3367 |
| 1 cell = 3 pg DNA; $10^6$ cells = 3 $\mu$g DNA | | | | | |

Table 3: Determination of E of PCR-Gag. Results of samples with low number of copies

| Sample | N Cop 10 $\mu$l PCR | Dilution factor | N Cop 25 $\mu$l PCR Tn | (Efficiency)[35] 100 | N Cop/$\mu$g DNA | N Cop/ million cells | N Cop/ million cells |
|---|---|---|---|---|---|---|---|
| 1 | 228900000 | 1 | 572250000 | 850500000 | 0.67284 | 2.01852 | 2.01852 |
| | | | | | | | |
| 2 | 868300000 | 1 | 21707500000 | 850500000 | 25.52322 | 76.56967 | 76.56967 |
| 3 | 37580 | 1 | 93950 | 850500000 | 0.00011 | 0.00033 | 0.00033 |
| 4 | 531.3 | 1 | 1328.25 | 850500000 | 0.00000 | 4.68519E-06 | 4.68519E- |
| 5 | 345100000 | 1 | 8627500000 | 850500000 | 10.14403 | 30.43210 | 30.43210 |
| 6 | 578600 | 2 | 2893000 | 850500000 | 0.00340 | 0.01020 | 0.01020 |
| 7 | 107000000 | 2 | 53500000000 | 850500000 | 62.90417 | 188.71254 | 188.71254 |
| 8 | 740700000 | 2 | 3.7035E+11 | 850500000 | 435.44974 | 1306.34934 | 1306.34934 |
| 9 | 232100000 | 2 | 1.1605E+11 | 850500000 | 136.44915 | 409.34748 | 409.34748 |
| 10 | 606.6 | 2 | 3033 | 850500000 | 0.00000 | 1.06984E-05 | 1.06984E- |
| 11 | 11440 | 2 | 57200 | 850500000 | 0.00007 | 0.00020 | 0.00020 |
| 12 | 978 | 2 | 4890 | 850500000 | 0.00001 | 1.72487E-05 | 1.72487E- |
| 13 | 1260 | 2 | 6300 | 850500000 | 0.00001 | 2.22222E-05 | 2.22222E- |
| 14 | 686 | 2 | 3430 | 850500000 | 0.00000 | 1.20988E-05 | 1.20988E- |
| 15 | 617 | 2 | 3085 | 850500000 | 0.00000 | 1.08818E-05 | 1.08818E- |

(continued)

| Sample | N Cop 10 $\mu$l PCR | Dilution factor | N Cop 25 $\mu$l PCR Tn | (Efficiency)[35] 100 | N Cop/$\mu$g DNA | N Cop/ million cells | N Cop/ million cells |
|---|---|---|---|---|---|---|---|
| 16 | 2167 | 2 | 10835 | 850500000 | 0.00001 | 3.82187E-05 | 3.82187E- |
| 17 | 457200 | 2 | 2286000 | 850500000 | 0.00269 | 0.00806 | 0.00806 |
| 18 | 221800000 | 2 | 11090000000 | 850500000 | 13.03939 | 39.11817 | 39.11817 |
| 19 | 117900000 | 2 | 58950000000 | 850500000 | 69.31217 | 207.93653 | 207.93653 |
| 20 | 432800000 | 2 | 21640000000 | 850500000 | 25.44386 | 76.33158 | 76.33158 |
| 21 | 330900000 | 2 | 16545000000 | 850500000 | 19.45326 | 58.35979 | 58.35979 |
| 22 | 17300000 | 2 | 86500000 | 850500000 | 0.10170 | 0.30511 | 0.30511 |

Example 6: Modification of the ultrasensitive method of nested quantitative PCR using a 2nd pair of primers different from the 1st.

For the purpose of avoiding deficiencies in the detection of the HIV genome in samples with small amount of DNA, and moreover to simplify the method and reduce the test duration, by processing all the samples equally without taking into account whether they might contain a high or low number of copies, another preferred embodiment of the invention was developed, comprising nested quantitative PCR.

The method comprise carrying out a nested PCR [8] [10] [11] using a 2nd pair of primers that binds in an internal region of the amplified product with the F-Gag/R-Gag primers of 222 bp (*Gag1*). This 2nd pair of primers, F2-Gag/R2-Gag, binds in the sequences that separate the oligonucleotides F-Gag - P1-Gag, and P2-Gag - R-Gag, respectively, but with a melting point (Tm) 10°C lower than the pair F-Gag/R-Gag, giving a product of 149 bp, *Gag2.* These differences in the Tm values make it possible to design a programme of "nested quantitative PCR" in a single capillary per sample with a reaction mixture containing the two pairs of primers and the probes. Each 20 $\mu$l of reaction mixture of LC-qPCR-Gag 2.0 contains from 10 to 200 ng of genomic DNA as template, 0.2 $\mu$M of each fluorescent probe P1-Gag and P2-Gag, 0.05 $\mu$M of the primers F-Gag and R-Gag, 0.5 $\mu$M of the primers F2-Gag and R2-Gag, 0.10 mM of dATP, 0.10 mM of dCTP, 0.10 mM of dTTP, 0.10 mM of dGTP, and 1 U of LightCycler FastStart DNA polymerase enzyme (Roche), 50 mM KCl, 3.5 mM MgCl$_2$, and 10 mM Tris-HCl (pH 8.3).

LC-qPCR-Gag 2.0 programme:

| | | |
|---|---|---|
| 1- Denaturing 1: | 95°C, 10 minutes | (20°C/s) |
| 2- Amplification 1, 15 cycles | 95°C, 15 seconds | (20°C/s) |
| | 65°C, 12 seconds | (20°C/s) |
| | 72°C, 12 seconds | (20°C/s) |
| 3- Denaturing 2: | 95°C, 2 minutes | (20°C/s) |
| 4- Amplification 2,30 cycles | 92°C, 4 seconds | (20°C/s) |
| *Fluorescence measurement* | 56°C, 10 seconds | (20°C/s) |
| | 72°C, 8 seconds | (20°C/s) |
| 5- Final denaturing: | 91°C, 10 seconds | (20°C/s) |
| | 60°C, 10 seconds | (20°C/s) |
| | 91°C, 1 second | (0.20°C/s) |
| 6- End of programme: | 40°C, 30 minutes. | |

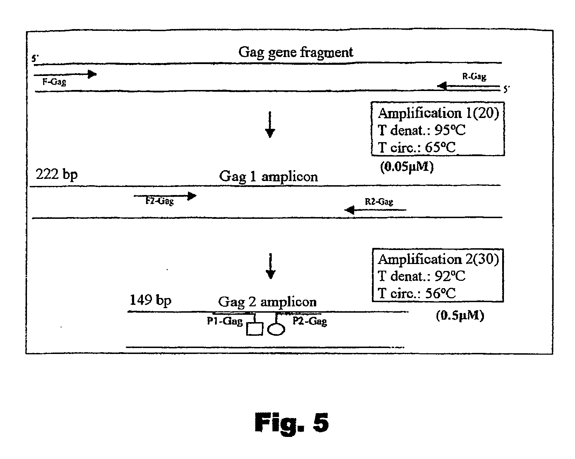

A first amplification is carried out, in which the F-Gag/R-Gag primers are hybridized in the template DNA exclusively, the F2-Gag and R2-Gag primers do not, even though their circle forming sequence is present, since their temperature of circle formation is 10°C lower than the pair F-Gag and R-Gag. After the 15 cycles of this first amplification, denaturing is effected at 95°C, which is the Tm of the product Gag1, thus the products obtained in Amplification 1 are denatured and are arranged for circle formation of the primers of Amplification 2. Then said amplification is initiated where the melting point is 92°C, at which only the product *Gag2* is denatured, but not *Gag1*, hence the product that is obtained in this amplification comes only from *Gag2,* thus different products originating from the same initial template DNA will not be counted. The products *Gag1* are not quantified even though the probes bind in them since the LightCycler thermal cycler is programmed for it, via its information application, so that fluorescence is only quantified during Amplification 2 (see diagram in Fig. 5).

To calculate Tm (melting point or denaturing temperature), a formula is applied that takes into account the number of bases G/C and A/T:

$$Tm = (wA+xT) * 2 + (yG+zC) * 4$$

where w, x, y, z denote the respective number of bases A, T, G, C in the sequence.

In the present invention the sequence was inserted in the program on the Internet page: http://www.basic.nwu.edu/biotools/oligocalc.html and the program itself calculated each value of Tm.

## Quantification and sensitivity

In order to be able to quantify the samples it is necessary to extrapolate the fluorescence obtained in them on a reference straight line for which the plasmid pBSK-Gag was used, which has the cloned amplification product *Gag1*.

The main problem that this method may present is the actual quantification of the samples, since the quantification of fluorescence takes place in the 2nd amplification, accordingly this fluorescence is associated with the product obtained in the 1st amplification, and not with the number of initial copies present in each sample. This problem is overcome by the actual quantification technique, on referring it to the reference straight line. The same occurs on this reference straight line as in the remaining samples, but on assigning them to the fluorescence obtained in the 2nd amplification the number of real copies present at the beginning of the PCR the fluorescence extrapolated on said reference straight line of the various samples will give, as the result, the initial copies present in each of them. Finally, what is achieved with this method is amplification of the fluorescence signal from each unit Gag, increasing the sensitivity of the method.

## Amplification 1, number of optimum cycles

The increase in sensitivity of the method is given by Amplification 1, but if excessive amplification occurs it could saturate and equalize the template DNA for Amplification 2 in all the samples, obtaining a similar number of counts in all the samples even though not all had similar quantities initially. To determine the number of cycles of amplification 1 that increase the sensitivity of the method if the template is saturated for the 2nd amplification, several LC-qPCR Gag 2.0 were carried out with different amounts of DNA from cells 8E5 as template, from 550 ng to 0.00055 ng, in various inter- and intra-test repetitions, concluding that the number of cycles at which greatest accuracy (least error) is obtained, even with small amounts of template DNA (0.55 ng per reaction) is 15 cycles (Table 4).

**Table 4:** Results of LC-qPCR Gag 2.0 with different numbers of cycles in Amplification 1: 20, 10 or 15, compared with the expected theoretical result. Amplification 2 always has 30 cycles.

| Summary of quantification Gag 2.0 in DNA from cells 8E5 | | | | | | | |
|---|---|---|---|---|---|---|---|
| Sample | Total DNA (ng) | Theoretical number of copies/cell | 20 cycles | 10 cycles | 15 cycles | 15 cycles | 15 cycles |
| E1 | 550 | 1 | 0.07 | 0.03 | - | 0.10 | 0.06 |
| E2 | 55 | 1 | 0.43 | 0.12 | 0.12 | 0.26 | 0.63 |
| E3 | 5.5 | 1 | 4.88 | 0.84 | 0.13 | 0.65 | 1.21 |
| E4 | 0.55 | 1 | 18.82 | 10.20 | 3.85 | 4.02 | 4.97 |
| E5 | 0.055 | 1 | 50.91 | 27.00 | 29.55 | 422.14 | 805.45 |
| E6 | 0.0055 | 1 | 2176.36 | 393.64 | 1619.09 | - | - |
| E7 | 0.00055 | 1 | 29727.27 | 2372.73 | 26681.82 | - | - |

## Reproducibility

The reproducibility of the results obtained with this method is assessed in Table 5. The variation in the intra-test duplicates is very low, except when the amount of template DNA is very low, starting from 0.55 ng, the inter-test variation is also very low, and does not exceed 1 unit up to 0.55 ng of template DNA, with smaller amounts the variability is very high, therefore from 5 ng to 500 ng has been taken as optimum amount of template DNA for this method.

**Table 5:** Data obtained in 2 different experiments carried out in the same conditions (amplification 1 of 15 cycles), with the same samples duplicated in each of them.

| | | Number of copies/Cell | | | |
|---|---|---|---|---|---|
| | Total DNA (ng) | exp 1 | average exp 1 | exp 2 | average exp 2 |
| E1a | 550 | 0.12 | 0.10 | 0.07 | 0.06 |
| E1b | 550 | 0.09 | | 0.06 | |
| E2a | 55 | 0.29 | 0.26 | 1.01 | 0.63 |
| E2b | 55 | 0.22 | | 0.26 | |
| E3a | 5.5 | 0.84 | 0.65 | 1.12 | 1.21 |
| E3b | 5.5 | 0.46 | | 1.29 | |
| E4a | 0.55 | 7.74 | 4.02 | 3.44 | 4.97 |
| E4b | 0.55 | 0.30 | | 6.51 | |
| E5a | 0.055 | 41.92 | 422.14 | 55.45 | 805.45 |
| E5b | 0.055 | 802.36 | | 1555.45 | |
| a, b: duplicates | | | | | |

The reference straight line is obtained as described in Example 3.

**References**

1. Guidelines for the Use of Antiretroviral Agents in HIV-1-Infected Adults and Adolescents. Developed by the Panel of Clinical Practices for Treatment of HIV Infection convened by the Department of Health and Human Services (DHHS). March 23, 2004.

2. Warner C. Greene and B. Matija Peterlin. 2002. Charting HIV's remarkable voyage through the cell:basis science as a passport to future therapy. Nature medicine. 8 (7).

3. Yupi Zhao, Min Yu, Johann W. Miller, and Ram Yogev. 2002. Quantification of HIV-1 proviral DNA by using Taqman Technology. Journal of Clinical Microbiology. 40(2). p. 675-678.

4. Una O'Doherty, W. J. Swiggard, Deepa Jeyakumar, David McGain and Michal H. Malin. 2002. A sensitive, quantitative assay for HIV-1 integration. Journal of Virology. 76(21). p 10942-10950.

5. Scott L. Butter, Mark S. T. Hansen and Frederic D. Bushman. 2001. Aquantitative assay for HIV DNA integration in vivo. Nature Medicine. 7(5).

6. L. G. Kostrikis, G. Toulomi, R. Karinicolas, and A. Hatzakis. 2002. Quantitation of HIV-1 DNA forms with the second template swich in peripheral blood cells predics disease progression independently of plasma RNA load. 76(20). p. 10099-10108.

7. Stefan Mener, Carl Witter, Kan-Ichi Nakagawara. 2001. Rapid Cycle Real Time PCR. Methods and Applications. Ed. Springer.

8. A. Rolfs, I. Schuller, U. Finckh, I. Weber-Rolfs. 1992. PCR: Clinical Diagnostics and Research. Ed. Springer.

9. I. A. Teo, J. W. Choi, J. Morlese, G. Taylor, S. Shaunak. 2002. LightCycler qPCR optimisation for low copy number target DNA. J Immunology Methods.

Description of the drawings

Fig. 1: PCR-Gag, 222 bp product. The bands obtained correspond to the product amplified with the primers F-Gag and R-Gag on the viral DNA.

Fig. 2: A) Electrophoresis in 1% agarose gel of the plasmid pBSK-Gag. The three conformers are assessed, 1 linear, 2 relaxed, 3 supercoiled. B) Electrophoresis in 2% agarose gel of the products from PCR-Gag with each conformer of the plasmid pBSK-Gag. The marker in the two gels is a reference standard in 100 bp steps in each band.

Fig. 3: Explanatory diagram of the system for quantification in samples with low number of copies.

Fig. 4: Graphs of the reference curve pBSK-Gag of LC-qPCR Gag from Table 1. Graphs a-f show the reference curves obtained in different experiments and demonstrate the reproducibility of the method, the coefficient of correlation and the error obtained in each one of the said experiments.

Fig. 5: Explanatory diagram of double nested quantitative PCR using different pairs of primers in each amplification step.

**Claims**

1. An in-vitro method of quantitative detection of DNA of the HIV virus that comprises a double amplification of viral DNA present in the sample using a double nested quantitative PCR carried out in such a way that a first pair of oliglonucleotide PCR primers SEQ ID NO: 1 and SEQ ID NO: 2, which bind specifically to the gag gene at positions 1260 to 1482 is used in the first amplification and a second pair of oliglonucleotide PCR primers with a melting point (Tm) different from that of the first pair of primers, which bind to the gag gene inside the fragment amplified by the first pair of primers **characterized in that** the amplification and the detection of the viral DNA are carried out in a single capillary per sample with a reaction mixture containing the two pairs of primers and the probes.

2. The method according to Claim 1, **characterized in that** a second pair of primers SEQ ID NO: 7 and SEQ ID NO: 8, binds specifically inside the gag gene fragment amplified by the first pair or primers between positions 1286 and 1435 of said gene.

3. The method according to any of the claims 1 or 2, **characterized in that** the sequence of nucleotide of one of the probes is represented by SEQ ID NO:5.

4. The method according to any of the claims 1 to 3, **characterized in that** the sequence of nucleotide of the second probe is represented by SEQ ID NO:6.

5. The method according to Claims 1 to 4, **characterized in that** the fist pair of primers are represented by SEQ ID NO:1 and SEQ ID NO:2, the second pair of primers are represented by SEQ ID NO:7 and SEQ ID NO:8 and the sequence of nucleotides of the probes are represented by SEQ ID NO:5 and SEQ ID NO:6.

6. The method according to anyone of the preceding claims, **caracterized in that** the probes are labelled with F.R.E.T. type fluorochromes, thereafter the number of copies of viral DNA present in the test sample is quantified by extrapolating the fluorescence obtained to a previously calibrated reference straight or curve.

7. Test kit for quantifying DNA of the HIV virus, **characterized in that** it comprises a reaction mixture in a single capillary that contains:

    i. at least one first pair of oligonucleotides PCR primers SEQ ID NO: 1 and SEQ ID NO: 2 which bind specifically to the gag gene between positions 1260 and 1482 of said gene,
    ii. at least one second pair of oligonucleotides PCR primers SEQ ID NO: 7 and SEQ ID NO: 8 with a melting point (Tm) different from that of the first pair of primers and which bind specifically to the gag gene inside the fragment determined by the first pairs or primers, that uses as template the product previously amplified by said first pair of primers,

and, jointly or separately in a reagent,

iii. a pair of oligonucleotides probes which bind specifically to the fragment of the gag gene comprised between the second pair of primers.

8. Test kit according to claim 7, **characterized in that** one of the oligonucleotide probes is represented by SEQ ID NO:5.

9. Test kit according to any of the claims 7 or 8, **characterized in that** the second oligonucleotide probe is represented by SEQ ID NO:6.

10. Test kit according to any of the claims 7 to 9, **characterized in that** the first pair of oligonucleotide primers is represented by SEQ ID NO:1 and SEQ ID NO:2, the second pair of oligonucleotide primers is represented by SEQ ID NO: 7 and SEQ ID NO:8 and the pair of oligonucleotide probes is represented by SEQ ID NO:5 and SEQ ID NO:6.

11. Test kit according to any of the claims 7 to 10, **characterized by** the pair of oligonucleotide probes being labelled with FRET-type fluorochromes.

**Patentansprüche**

1. Ein In-vitro-Verfahren zur quantitativen Erfassung von DNA des HIV-Virus, das die zweifache Amplifikation der in der Probe vorhandenen viralen DNA umfasst, wobei eine zweifach verschachtelte PCR angewendet wird, welches in der Weise durchgeführt wird, dass ein erstes Paar von Oligonukleotid-PCR-Primeren SEQ ID NO 1 und SEQ ID NO 2, welches insbesondere mit dem gag-Gen an den Positionen 1260 bis 1482 verbunden ist, bei der ersten Amplifikation benutzt wird, und ein zweites Paar von Oligonukleotid-PCR-Primeren mit einem Schmelzpunkt (Tm), der von jenem des ersten Paares von Primeren abweicht, welches mit dem gag-Gen innerhalb des durch das erste Paar von Primeren amplifizierten Fragments verbunden ist, **dadurch gekennzeichnet, dass** die Amplifikation und die Erfassung der viralen DNA in einem einzeigen Kapillargefäß je Probe durchgeführt wird, mit einer Reaktionsmischung, die die beiden Paare von Primeren und die Proben enthält.

2. Das Verfahren nach Anspruch 1, **dadurch gekennzeichnet, dass** ein zweites Paar von Primeren SEQ ID NO 7 und SEQ ID NO 8 insbesondere innerhalb des durch das erste Paar von Primeren amplifizierten gag-Gen-Fragments an den Positionen 1286 bis 1435 des besagten Gens verbunden ist.

3. Das Verfahren nach einem der Ansprüche 1 oder 2, **dadurch gekennzeichnet, dass** die Nukleotidsequenz einer der Proben durch die SEQ ID NO 5 dargestellt wird.

4. Das Verfahren nach einem der Ansprüche 1 bis 3, **dadurch gekennzeichnet, dass** die Nukleotidsequenz der zweiten Probe durch die SEQ ID NO 6 dargestellt wird.

5. Das Verfahren nach einem der Ansprüche 1 bis 4, **dadurch gekennzeichnet, dass** das erste Paar der Primere durch die SEQ ID NO 1 und SEQ ID NO 2 dargestellt wird, das zweite Paar der Primere durch die SEQ ID NO 7 und SEQ ID NO 8 dargestellt wird und die Nukleotidsequenz der Proben durch die SEQ ID NO 5 und SEQ ID NO 6 dargestellt wird.

6. Das Verfahren nach einem der vorhergehenden Ansprüche, **dadurch gekennzeichnet, dass** die Proben mit Fluorochromen des Typs FRET markiert werden, wonach die Anzahl der Exemplare der in der Testprobe vorhandenen viralen DNA quantifiziert wird, indem die Fluoreszenz extrapoliert wird, welche durch eine vorher kalibrierte Referenzgerade oder -kurve erhalten wurde.

7. Testkit zur Quantifizierung von DNA des HIV-Virus, **dadurch gekennzeichnet, dass** es eine Reaktionsmischung in einem einzigen Kapillargefäß umfasst, welche Folgendes umfasst:

i. mindestens ein erstes Paar von Oligonukleotid-PCR-Primeren SEQ ID NO 1 und SEQ ID NO 2, welches insbesondere mit dem gag-Gen an den Positionen 1260 bis 1482 des besagten Gens verbunden ist,

ii. mindestens ein zweitens Paar von Oligonukleotid-PCR-Primeren SEQ ID NO 7 und SEQ ID NO 8 mit einem Schmelzpunkt (Tm), der von jenem des ersten Paares von Primeren abweicht, welches insbesondere mit dem gag-Gen innerhalb des durch das erste Paar von Primeren bestimmten Fragments verbunden ist, welches als Vorlage das vorher durch dieses erste Paar von Primeren amplifizierte Produkt verwendet, und zusammen oder getrennt in einem Reagens,

iii. ein Paar von Ongonukleotidproben, welches insbesondere mit dem Fragment des gag-Gens verbunden ist, welches zwischen dem zweiten Paar von Primeren umfasst ist.

8. Testkit nach Anspruch 7, **dadurch gekennzeichnet, dass** eine der Oligonukleotidproben durch die (SEQ ID NO 5 dargestellt wird.

9. Testkit nach einem der Ansprüche 7 oder 8, **dadurch gekennzeichnet dass** die zweite Oligonukleotidprobe durch die SEQ ID NO 6 dargestellt wird.

10. Testkit nach einem der Ansprüche 7 bis 9, **dadurch gekennzeichnet, dass** das erste Paar der Oligonukleotidprimere durch die SEQ ID NO 1 und SEQ ID NO 2 dargestellt wird, das zweite Paar der Oligonukleotidprimere durch die SEQ ID NO 7 und SEQ ID NO 8 dargestellt wird und das Paar der Oligonukleotidproben durch die SEQ ID NO 5 und SEQ ID NO 6 dargestellt wird.

11. Testkit nach einem der Ansprüche 7 bis 10, **dadurch gekennzeichnet, dass** die Oligonukleotidproben mit Fluorochromen des Typs FRET markiert werden.

**Revendications**

1. Méthode in vitro de détection quantitative de l'ADN du virus de l'immunodéficience humaine comprenant une double amplification de l'ADN viral présent dans l'échantillon réalisée au moyen d'une PCR double quantitative menée de sorte qu'une première paire d'amorces oligonucléotidiques de PCR SEQ ID NO: 1 et SEO ID NO: 2 qui se lie spécifiquement au gène gag aux positions 1260 et 1482 est utilisée pour la première amplification et qu'une seconde paire d'amorces oligonucléotidiques de PCR dont le point de fusion (Tm) diffère de celui de la première paire d'amorces se lie au gène gag à l'intérieur du fragment amplifié par la première paire d'amorces, **caractérisée par le fait que** l'amplification et la détection de l'ADN viral sont effectuées dans un seul capillaire par échantillon avec un mélange de réaction contenant les deux paires d'amorces et les sondes.

2. Méthode selon la Revendication 1, **caractérisée par le fait qu'**une seconde paire d'amorces SEQ ID NO: 7 et SEQ ID NO: 8 se lie spécifiquement à l'intérieur du fragment du gène gag amplifié par la première paire d'amorces entre les positions 1286 et 1435 dudit gène.

3. Méthode selon n'importe laquelle des Revendications 1 ou 2, **caractérisée par le fait que** la séquence de nucléotides d'une des sondes est représentée par SEQ ID NO: 5.

4. Méthode selon n'importe laquelle des Revendications 1 à 3, **caractérisée par le fait que** la séquence de nucléotides de la seconde sonde est représentée par SEQ ID NO: 6.

5. Méthode selon les Revendications 1 à 4, **caractérisée par le fait que** la première paire d'amorces est représentée par SEQ ID NO: 1 et SEQ ID NO: 2, la seconde paire d'amorces par SEQ ID NO: 7 et SEQ ID NO: 8 et les séquences de nucléotides des sondes par SEQ ID NO: 5 et SEQ ID NO: 6.

6. Méthode selon n'importe laquelle des Revendications précédentes, **caractérisée par le fait que** les sondes sont marquées par des fluorochromes de type FRET, à la suite de quoi le nombre de copies d'ADN viral présentes dans l'échantillon de l'essai est quantifié en extrapolant la fluorescence obtenue grâce à une droite ou une courbe de référence calibrée au préalable.

7. Kit d'essai pour la quantification de l'ADN du virus de l'immunodéficience humaine, **caractérisé par le fait qu'**il comprend un mélange de réaction dans un capillaire unique contenant :

i. au moins une première paire d'amorces oligonucléotidiques de PCR SEQ ID NO: 1 et SEQ ID NO: 2, qui se lie spécifiquement au gène gag entre les positions 1260 et 1482 dudit gène,
ii. au moins une seconde paire d'amorces oligonucléotidiques de PCR SEQ ID NO: 7 et SEQ ID NO: 8 dont le point de fusion (Tm) diffère de celui de la première paire d'amorces et qui se lie spécifiquement au gène gag à l'intérieur du fragment déterminé par la première paire d'amorces, utilisant comme motrice le produit préalablement amplifié par ladite première paire d'amorces,

et, conjointement ou séparément dans un réactif,

iii. une paire de sondes oligonucléotidiques qui se lie spécifiquement au fragment du gène gag compris à l'intérieur de la seconde paire d'amorces.

8. Kit d'essai selon la Revendication 7, **caractérisé par le fait qu'**une des sondes oligonucléotidiques est répréseentée par SEQ ID NO: 5.

9. Kit d'essai selon n'importe quelle des Revendications 7 ou 8, **caractérisé par le fait que** la seconde sonde oligonucléotidique est représentée par SEQ ID NO: 6.

10. Kit d'essai selon n'importe laquelle des Revendications 7 à 9, **caractérisé par le fait que** la première paire d'amorces oligonucléotidiques est représentée par SEQ ID NO: 1 et SEQ ID NO: 2, la seconde paire d'amorces oligonucléotidiques par SEQ ID NO: 7 et SEQ ID NO: 8 et la, paire de sondes oligonocléotidiques par SEQ ID NO: 5 et SEQ ID NO: 6.

11. Kit d'essai selon n'importe laquelle des Revendications 7 à 10, **caractérisé par le fait que** la paire de sondes oligonucléotidiques est marquée par des fluorochromes de type FRET.

**Fig. 1**

Extraction
Conformers
and PCR-Gag

600 bp—
300 bp—

**Fig. 2**

**Fig. 3**

**Fig. 4**

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Yourno et al.** *J. Clinical Microbiol.,* 1992, vol. 30, 2887-2892
- **Warner C. Greene ; B. Matija Peterlin.** Charting HIV's remarkable voyage through the cell:basis science as a passport to future therapy. *Nature medicine,* 2002, vol. 8 (7
- **Yupi Zhao ; Min Yu ; Johann W. Miller ; Ram Yogev.** Quantification of HIV-1 proviral DNA by using Taqman Technology. *Journal of Clinical Microbiology,* 2002, vol. 40 (2), 675-678
- **Una O'Doherty ; W. J. Swiggard ; Deepa Jeyakumar ; David McGain ; Michal H. Malin.** A sensitive, quantitative assay for HIV-1 integration. *Journal of Virology,* 2002, vol. 76 (21), 10942-10950
- **Scott L. Butter ; Mark S. T. Hansen ; Frederic D. Bushman.** Aquantitative assay for HIV DNA integration in vivo. *Nature Medicine,* 2001, vol. 7 (5
- **L. G. Kostrikis ; G. Toulomi ; R. Karinicolas ; A. Hatzakis.** Quantitation of HIV-1 DNA forms with the second template swich in peripheral blood cells predics disease progression independently of plasma. *RNA load,* 2002, vol. 76 (20), 10099-10108
- **Stefan Mener ; Carl Witter ; Kan-Ichi Nakagawara.** Rapid Cycle Real Time PCR. Methods and Applications. Springer, 2001
- **A. Rolfs ; I. Schuller ; U. Finckh ; I. Weber-Rolfs.** PCR: Clinical Diagnostics and Research. 1992
- **I. A. Teo ; J. W. Choi ; J. Morlese ; G. Taylor ; S. Shaunak.** LightCycler qPCR optimisation for low copy number target DNA. *J Immunology Methods,* 2002

Sequence listing

<110> Genomadrid
<120> Method of in-vitro detection and quantification of HIV DNA by quantitative PCR
<130> P-100143
<160> 6
<210> 1
<211> 26
<213> Artificial sequence
<220>
<221> Primer
<223> F-Gag
<400> TAGTAGAAGA GAAGGCTTTC AGCCCA

<210> 2
<211> 24
<213> Artificial sequence
<220>
<221> Primer
<223> R-Gag
<400> TTGGTTCTCT CATCTGGCCT GGTG

<210> 3
<211> 36
<213> Artificial sequence
<220>
<221> Primer
<223> F-Gag-Bam
<400> CGGGATCCCG TAGTAGAAGA GAAGGCTTTC AGCCCA

<210> 4
<211> 34
<213> Artificial sequence
<220>
<221> Primer
<223> R-Gag-Eco
<400> CGGAATTCCG TTGGTTCTCT CATCTGGCCT GGTG

<210> 5
<211> 30
<213> Artificial sequence
<220>
<221> Probe
<223> P1-Gag
<400> GAGCCACCCC ACAAGATTTA AACACCATGT

<210> 6

<211> 29
<213> Artificial sequence
<220>
<221> Probe
<223> P2-Gag
<400> AACACAGTGG GGGGACATCA AGCAGCCAT

<210> 7
<211> 22
<213> Artificial sequence
<220>
<221> Primer
<223> F2-Gag
<400> GAAGTAATAC CCATGTTTTC AG

<210> 8
<211> 21
<213> Artificial sequence
<220>
<221> Primer
<223> R2-Gag
<400> TGCAGAATGG GATAGATTGC A